# EUROPEAN PATENT APPLICATION

(11) **EP 1 679 038 A2**
(43) Date of publication of application: **12.07.2006**
(21) Application number: 05022972.3
(22) Date of filing: 21.10.2005
(51) Int. Cl.: A61B 8/14

(54) **Ultrasound diagnostic system and method of forming arbitrary m-mode images**

(30) Priority: 05.01.2005 KR 2005000709
(71) Applicant: MEDISON CO., LTD., Kangwon-do 250-870 (KR)
(72) Inventor: Kim, Jeong Hwan, Guro-gu Seoul 152-767 (KR)
(74) Representative: Lorenz, Werner

(57) **Abstract**

The present application relates to an ultrasound diagnostic system and a method of forming arbitrary M-mode ultrasound images. The ultrasound diagnostic system (100) includes: a probe (110) for transmitting ultrasound signals toward a desired part of a target object and receiving the ultrasound signals reflected from the desired part; a scan converter (150) for converting an ultrasound image data into a B-mode image data, the ultrasound image data being obtained based on the ultrasound signal received from the probe; an input unit (180) for receiving an arbitrary M-mode scan line set by a user; an arbitrary M-mode processor (171) for generating an arbitrary M-mode image data corresponding to the arbitrary M-mode scan line; and a display unit (190) for displaying at least one of the B-mode image data, the M-mode image data and the arbitrary M-mode scan line. In accordance with the present application, the biological information of the target object can be observed and diagnosed from an arbitrary direction and path, regardless of the direction of the ultrasound signals. Additionally, the arbitrary M-mode image, which is more similar to the real image, can be provided for the user by generating the arbitrary M-mode images corresponding to the arbitrary M-mode scan line by using ultrasound image data before scan-conversion.

## Description

The present invention generally relates to an ultrasound diagnostic system and a method of displaying arbitrary M-mode images, and more particularly to an ultrasound diagnostic system and a method of forming arbitrary M-mode images for observing a transition trend of an ultrasound image information in a specific line of the ultrasound diagnostic image.

An ultrasound diagnostic system is widely used for non-invasively obtaining tomographic images of soft tissue and blood flow within a human body. In such a system, the tomographic images are formed through the process of (1) transmitting an ultrasound signal from a surface of the human body toward a desired part within the human body, (2) receiving the ultrasound signal reflected from within the human body, and (3) performing signal processing upon the received ultrasound signal (echo signal). Compared to other medical imaging systems (e.g., X-ray diagnostic system, X-ray Computerized Tomography (CT) scanner, Magnetic Resonance Imaging (MRI) system, nuclear medicine diagnostic system, etc.), the ultrasound diagnostic system is relatively small in size and inexpensive. Further, it is capable of displaying images in real-time and is highly safe from exposure to X-ray radiation, etc. Thus, the ultrasound diagnostic system is extensively used to diagnose the heart, the abdomen and the urinary organs in the fields of obstetrics, gynecology, etc.

In order to meet such diagnostic needs, the ultrasound diagnostic system has been provided with certain functions relating to basic image display modes for examination and diagnosis (e.g., M-mode, B-mode, Continuous Wave (CW) Doppler mode, Pulsed Doppler (PD) mode, Color Flow Mapping (CFM) mode, etc.), which are based on the ultrasonic wave pulse reflection method.

Among the image display modes mentioned above, the B-mode is used to display cross-sectional images within the human body by electronically converting the strength of the ultrasound echo signals into intensity-modulated dots on a display unit. The M-mode is used to display how the biological information of the target object, which is to be inspected (e.g., brightness), varies with time in a cross-sectional image of the target object (i.e., B-mode image).

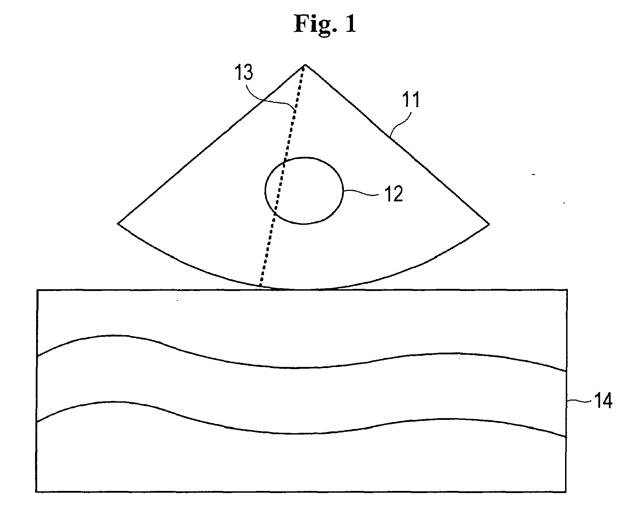

The B-mode and M-mode images are described more specifically by referring to Fig. 1, wherein: reference numeral 11 indicates a cross-sectional image of the target object, that is, the B-mode image; reference numeral 12 points to an interface surface of the target object; reference numeral 13 indicates an M-mode scan line; and reference numeral 14 indicates the biological information image of the target object portion corresponding to the M-mode scan line 13, which varies with time.

However, the conventional ultrasound diagnostic system sets a specific single scan line out of a plurality of scan lines used to form the B-mode image as the M-mode scan line *(see* reference numeral 13 shown in Fig. 1). Thus, it has drawbacks in that the M-mode image is obtained only in a proceeding direction of the ultrasound signals. Accordingly, a user must artfully change the proceeding direction of the ultrasound signals in order to diagnose the desired parts of the target object, which prolongs the diagnostic time.

It is, therefore, an objective of the present invention to provide an ultrasound diagnostic system and a method of forming arbitrary M-mode images, which can observe and diagnose the biological information of the target object at an arbitrary direction and path, regardless of the proceeding direction of ultrasound signals.

In accordance with one aspect of the present invention, there is provided an ultrasound diagnostic system comprising: a probe for transmitting ultrasound signals toward a desired part of a target object and receiving the ultrasound signals reflected from the desired part; a scan converter for converting an ultrasound image data into a B-mode image data, the ultrasound image data being obtained based on the ultrasound signal received from the probe; an input unit for receiving an arbitrary M-mode scan line set by a user; an arbitrary M-mode processor for generating an arbitrary M-mode image data corresponding to the arbitrary M-mode scan line; and a display unit for displaying at least one of the B-mode image data, the M-mode image data and the arbitrary M-mode scan line.

In accordance with another aspect of the present invention, there is provided a method of displaying an arbitrary M-mode image, comprising the steps of: a) transmitting an ultrasound signal from a body surface of a target object toward a desired part and receiving the ultrasound signal reflected from the desired part; b) converting the received ultrasound signal into an ultrasound image data; c) converting the ultrasound image data into a B-mode image to display the B-mode image; d) receiving an arbitrary M-mode scan line on the B-mode image; e) forming an arbitrary M-mode image data corresponding to the arbitrary M-mode scan line; and f) displaying the arbitrary M-mode image data.

In accordance with yet another aspect of the present invention, there is provided a method of displaying an arbitrary ultrasound image by using an ultrasound diagnostic system including a probe for acquiring ultrasound signals from a plurality of scan lines, an image signal processor, a scan converter, an input unit, an arbitrary M-mode processor, and a display unit, the method comprising the steps of: a) obtaining the ultrasound signals from the probe; b) converting the ultrasound signals into an ultrasound image data by the image signal processor; c) converting the ultrasound image data into a B-mode image by the scan converter; d) displaying the B-mode image at the display unit; e) receiving an arbitrary M-mode scan line on the B-mode image from the input unit; f) forming an arbitrary M-mode image data corresponding to the arbitrary M-mode scan line by the arbitrary M-mode processor; and g) displaying the arbitrary M-mode image data at the display unit.

The above and other objects and features of the present invention will become apparent from the following description of preferred embodiments given in conjunction with the accompanying drawings, in which:
Fig. 1 is a conceptual drawing showing an example of conventional B-mode and M-mode images;
Fig. 2 is a block diagram schematically showing an ultrasound diagnostic system constructed in accordance with one embodiment of the present invention;
Fig. 3A is an explanatory view showing an ultrasound diagnostic image data on an x-y coordinate system before scan-conversion in accordance with one embodiment of the present invention;
Fig. 3B is an explanatory view showing an ultrasound diagnostic image data on an x-y coordinate system after scan-conversion in accordance with one embodiment of the present invention;
Fig. 4A is an explanatory view showing an arbitrary M-mode scan line set on a B-mode image in accordance with one embodiment of the present invention.
Fig. 4B is an explanatory view showing an arbitrary M-mode scan line on the x-y coordinate system before scan-conversion in accordance with one embodiment of the present invention.
Fig. 5 is an explanatory view showing an arbitrary M-mode image data corresponding to an arbitrary M-mode scan line in accordance with one embodiment of the present invention.
Fig. 6 is an explanatory view showing a B-mode image and an arbitrary M-mode image in accordance with one embodiment of the present invention.
Fig. 7 is a flow chart showing the procedure of forming an arbitrary M-mode image in accordance with one embodiment of the present invention.

Hereinafter, a preferred embodiment of the present invention will be described below with reference to accompanying drawings.

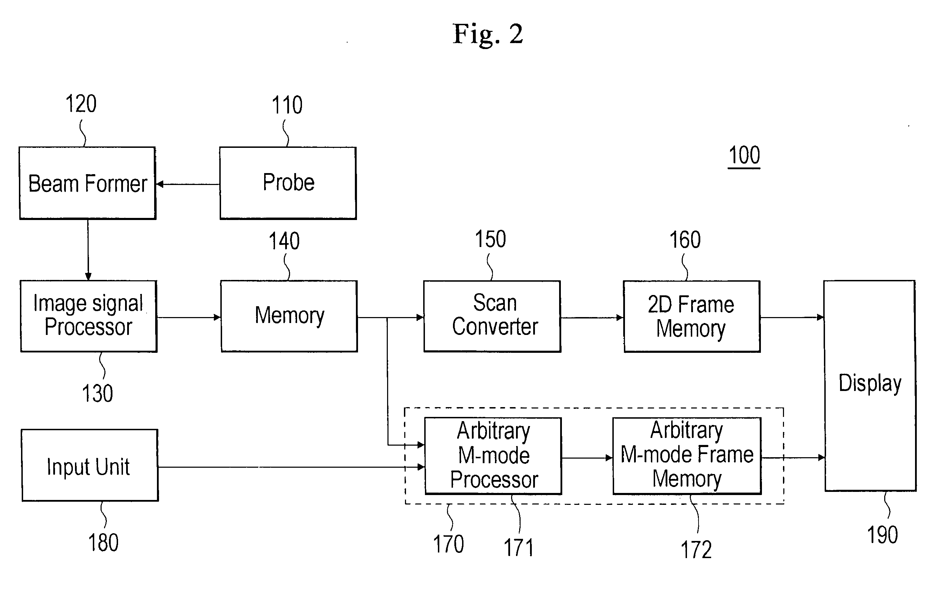

Fig. 2 is a block diagram schematically showing an ultrasound diagnostic system, which is constructed in accordance with one embodiment of the present invention.

Referring to Fig. 2, the ultrasound diagnostic system 100 includes a probe 110, a beam former 120, an image signal processor 130, a memory 140, a scan converter 150, a 2D frame memory 160, an arbitrary M-mode unit 170, a displaying device 190 and an input unit 180, wherein the arbitrary M-mode unit 170 has an arbitrary M-mode processor 171 and an M-mode frame memory 172.

A probe 110 includes a plurality of 1D or 2D transducers (not shown). The probe 110 transmits ultrasound signals to organs and receives the ultrasound signals reflected from the organs.

A beam former 120 adjusts a driving time of each transducer of a probe 110 when the probe 110 transmits the ultrasound signals, thereby focusing the ultrasound signals to desired positions. Further, the beam former 120 focuses the received ultrasound signals by applying a time delay to each of the received ultrasound signals, considering that each of the reflected ultrasound signals reaches each transducer of the probe 110 with different time interval.

An image signal processor 130, e.g., Digital Signal Processor (DSP), performs an envelope detection process in which the magnitude of ultrasound signals is detected upon the ultrasound signals focused by the beam former 120 so as to form an ultrasound diagnostic image data. The image signal processor 130 performs a log magnitude process to magnify the relative brightness difference of dark regions and reduces the relative brightness difference for bright regions in ultrasound images. The image signal processor 130 performs the log magnitude process to resolve the problem that the B-mode image, which is obtained based on the ultrasound signal focused by the beam former 120, has a large brightness difference between the dark region and the bright region so that only the bright region or only the dark region is displayed clearly.

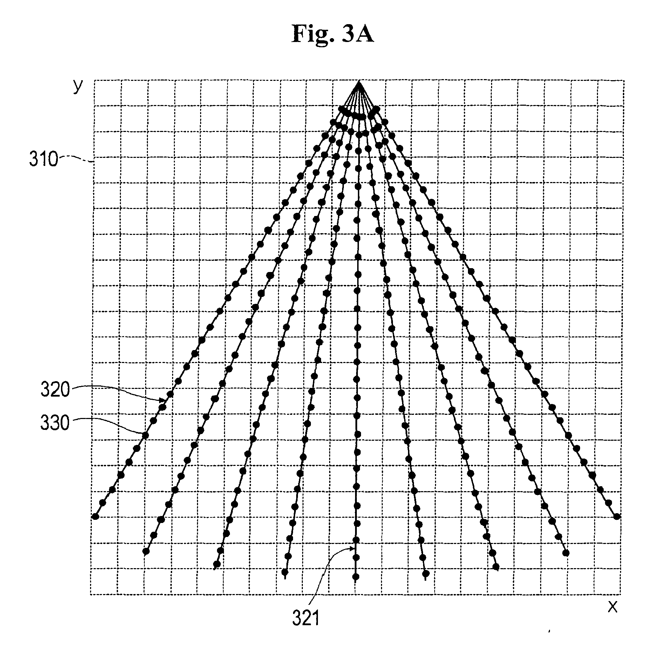

A memory 140 stores the ultrasound image data outputted from the image signal processor 130. Referring to Fig. 3A, each scan line 320 has a plurality of points 330. The image signal processor 130 forms ultrasound image data based on the position information and data (hereinafter referred to as "point data") of each point 330. That is, the ultrasound image data includes the coordinate information of a x-y coordinate system of each point 330, the angle information between each scan line 320 and vertical scan line 321, and the point data of each point 330. The above-mentioned ultrasound image data are stored in the memory 140 for each time.

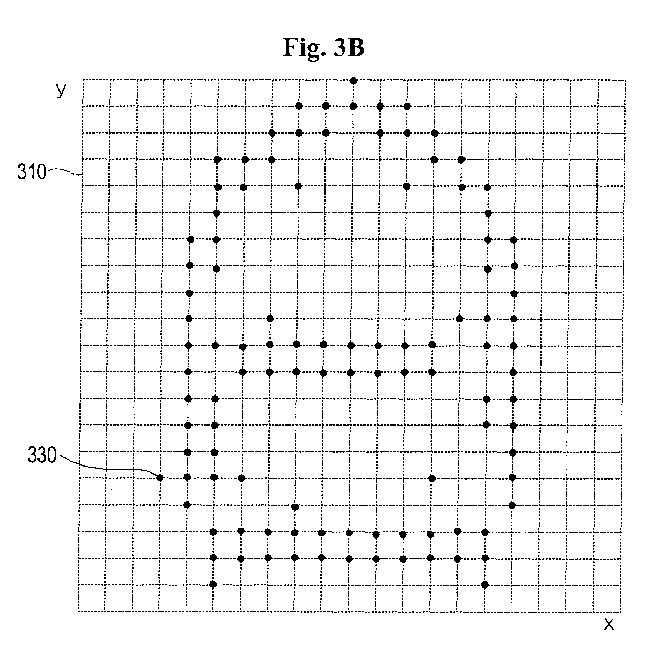

A scan converter 150 reads the ultrasound image data from the memory 140, converts the ultrasound image data into the ultrasound image data of a B-mode image format (hereinafter referred to as "B-mode image data"), and stores the B-mode image data into the 2D frame memory 160. Fig. 3B shows the ultrasound image data scan-converted by the scan converter 150 on an x-y coordinate system.

An input unit 180 receives the user's instruction for setting an arbitrary M-mode scan line on B-mode images displayed on a display device 190. The input unit 170 includes a keyboard, a track ball and a touch panel installed to the ultrasound diagnostic system 100. For, example, the user can select an arbitrary M-mode on/off setting menu using the touch panel, set and move the arbitrary M-mode scan line using the track ball, and move the angle and position of the arbitrary M-mode scan line using a knob of the keyboard.

An M-mode unit 170 includes an arbitrary M-mode processor 171 and an arbitrary M-mode frame memory 172. The arbitrary M-mode processor 171 reads the ultrasound image data corresponding to the arbitrary M-mode scan line and forms ultrasound image data of an arbitrary M-mode image format (hereinafter referred to as "arbitrary M-mode image data") based on the read ultrasound image data. The arbitrary M-mode frame memory 172 stores the arbitrary M-mode image data outputted from the arbitrary M-mode processor 171.

The display device 190 displays the B-mode image and the arbitrary M-mode image based on the image data stored in the 2D frame memory 160 and the data stored in the arbitrary M-mode frame memory 172, respectively.

The 2D frame memory 160 and the M-mode frame memory 172 can be implemented in a single memory.

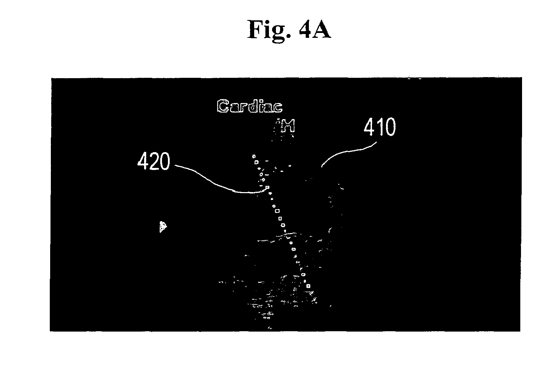

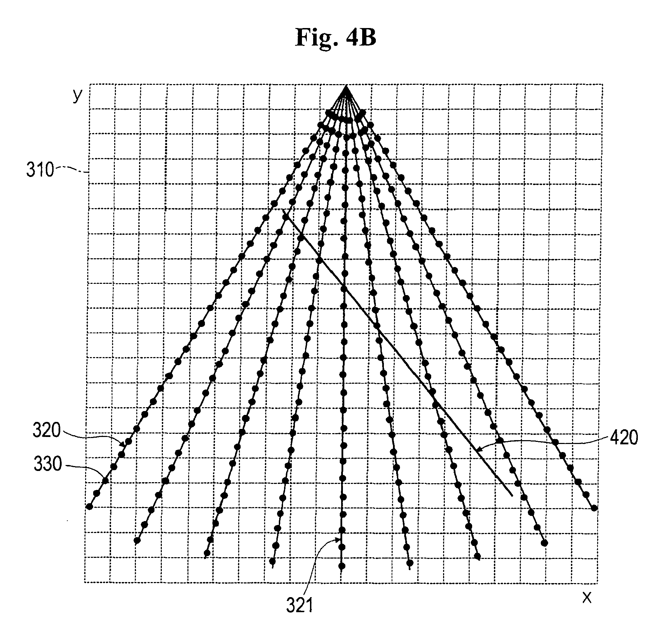

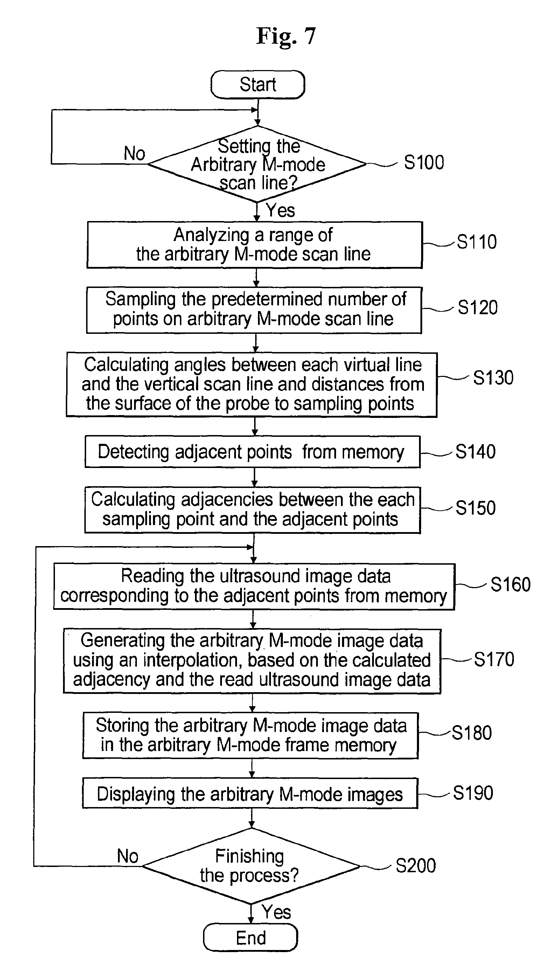

Figs. 3A to 7 describe the function of the arbitrary M-mode of the ultrasound image system 100 shown in Fig. 2 and the method of displaying the arbitrary M-mode image in accordance with the present invention.

When the user sets an arbitrary M-mode scan line 420 to designate a portion to be observed on the B-mode images displayed on the display device 190 by the input unit 180 at step S100, the arbitrary M-mode processor 171 in an arbitrary M-mode unit 170 analyzes the position and angle of the arbitrary M-mode scan line 420 set by the user. Accordingly, it generates the arbitrary M-mode image data corresponding to the arbitrary M-mode scan line 420 based on the ultrasound image data stored in memory 140.

To generate the arbitrary M-mode image data, the arbitrary M-mode processor 171 performs the following procedures. First, the arbitrary M-mode processor 171 analyzes the range of the arbitrary M-mode scan line 420 set by the user at step S110.
It then samples a predetermined number, and more preferably hundreds of points on the arbitrary M-mode scan line 420 at step S120. The arbitrary M-mode processor 171 sets virtual lines that pass through each sampling point and face toward the probe 110, and calculates the angles between each virtual line and the vertical scan line 321 and the distances from the surface of the probe 110 to the sampling points at step 130. Next, the arbitrary M-mode processor 171 detects a plurality of points (hereinafter referred to as "adjacent points") on M-mode scan lines 320 adjacent to each sampling point from memory 140 at step S 140. The arbitrary M-mode processor 171 calculates the adjacencies between each sampling point and the adjacent points at step S150.
The arbitrary M-mode processor 171 reads the ultrasound image data corresponding to the adjacent points from memory 140 at step S160. The arbitrary M-mode processor 171 generates the ultrasound image data (that is, the arbitrary M-mode image data) of each sampling point by using an interpolation based on the calculated adjacency and the read ultrasound image data at step S170. The arbitrary M-mode processor 171 stores the arbitrary M-mode image data in arbitrary M-mode frame memory 182 at step S180.

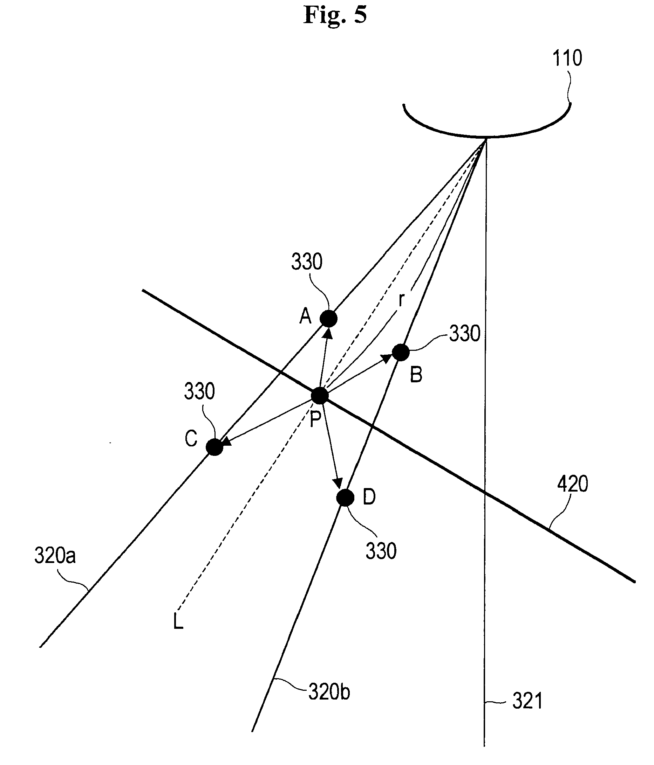

Referring to Fig. 5, the method of generating the arbitrary M-mode image data by the arbitrary M-mode processor 171 will be described in detail.

In Fig. 5, point P indicates an arbitrary point on an arbitrary M-mode scan line 420 (i.e., a sampling point) and points A to D indicate points on the arbitrary M-mode scan lines 320 adjacent to the point P (i.e., the adjacent points). More specifically, the points A, B, C and D are on the arbitrary M-mode scan lines 320a and 320b, which are adjacent to the point P on the left and right side, respectively. Further, the points A and C indicate the adjacent points on the arbitrary M-mode scan line 320a, while the points B and D indicate the adjacent points on the arbitrary M-mode scan line 320b. At step S 130, the arbitrary M-mode processor 171 calculates the angle between a vertical scan line 321 and a virtual line L that passes on the point P and faces toward the center of the probe 110, and also calculates the distance R from the probe 110 to the point P. At step S140, the arbitrary M-mode processor 171 detects the adjacent points A to D. At step S 150, the arbitrary M-mode processor 171 calculates the angles between the virtual line L and the adjacent scan lines 320a and 320b, calculates the distances from the probe 110 to each point A to D, and calculates the adjacencies between the sampling point P and the adj acent points A to D. At step S160, the arbitrary M-mode processor 171 reads the ultrasound image data corresponding to the adjacent points A to D from memory 140. At step S 170, the arbitrary M-mode processor 171 generates the arbitrary M-mode image data of the point P using an interpolation based on the calculated adjacencies and the read ultrasound image data. The arbitrary M-mode processor 171 calculates the arbitrary M-mode image data of all the sampling points on the arbitrary M-mode scan line through the aforementioned procedure.

After the step 170, the arbitrary M-mode image data generated by the arbitrary M-mode processor 171 are stored in the arbitrary M-mode frame memory 182 at step S180.

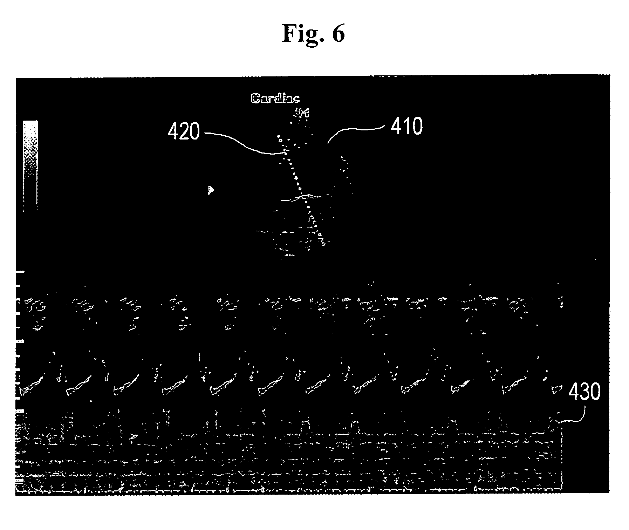

As shown in Fig. 6, the display device 190 displays the arbitrary M-mode image 430 together with the B-mode image 410 from the 2D frame memory 160 and the arbitrary M-mode scan line 420 set by the user through the input unit 180 at step S 190.

With the elapse of time, the ultrasound image data are continuously stored in the memory 140. Accordingly, the arbitrary M-mode processor 171 determines whether or not the arbitrary M-mode image is continuously displayed at step S200. If it is determined that the arbitrary M-mode image is continuously displayed, the arbitrary M-mode processor 171 performs the steps S 160 to S 180 to continuously display the arbitrary M-mode image on the arbitrary M-mode scan line, which may show the transition trend of the target object with the elapse of time. Further, when the displaying frequency of the arbitrary M-mode image is 120 Hz, the arbitrary M-mode processor 171 generates one arbitrary M-mode frame per 1/120 second, that is, 120 frames of the arbitrary M-mode image data are generated per second.
In accordance with the aforementioned embodiment of the present invention, each of the steps S 130 to S 180 is applied to all sampling points after sampling a predetermined number of points on the arbitrary M-mode scan line. However, the arbitrary M-mode ultrasound image data can be obtained by repeating steps S 130 to S180 as many times as the number of the sampling points.

As described above, in accordance with the present invention, the biological information of the target object can be observed and diagnosed from an arbitrary direction and path, regardless of the direction of the ultrasound signals. Additionally, the arbitrary M-mode image, which is more similar to the real image, can be provided for the user by generating the arbitrary M-mode images corresponding to the arbitrary M-mode scan line by using ultrasound image data before scan-conversion.

While the present invention has been described and illustrated with respect to a preferred embodiment of the invention, it will be apparent to those skilled in the art that variations and modifications are possible without deviating from the broad principles and teachings of the present invention which should be limited solely by the scope of the claims appended hereto.

## Claims

1. An ultrasound diagnostic system, comprising:
a probe for transmitting ultrasound signals toward a desired part of a target object and receiving the ultrasound signals reflected from the desired part;
a scan converter for converting an ultrasound image data into a B-mode image data, the ultrasound image data being obtained based on the ultrasound signal received from the probe;
an input unit for receiving an arbitrary M-mode scan line set by a user;
an arbitrary M-mode processor for generating an arbitrary M-mode image data corresponding to the arbitrary M-mode scan line; and
a display unit for displaying at least one of the B-mode image data, the M-mode image data and the arbitrary M-mode scan line.

2. The ultrasound diagnostic system of Claim 1, wherein the ultrasound diagnostic system further comprises at least one memory for storing the ultrasound image data and the arbitrary M-mode image data.

3. A method of displaying an arbitrary M-mode image, comprising the steps of:
a) transmitting an ultrasound signal from a body surface of a target object toward a desired part and receiving the ultrasound signal reflected from the desired part;
b) converting the received ultrasound signal into an ultrasound image data;
c) converting the ultrasound image data into a B-mode image to display the B-mode image;
d) receiving an arbitrary M-mode scan line on the B-mode image;
e) forming an arbitrary M-mode image data corresponding to the arbitrary M-mode scan line; and
f) displaying the arbitrary M-mode image data.

4. The method of Claim 3, wherein the step e) includes the steps of:
e1) sampling a predetermined number of points on the arbitrary M-mode scan line;
e2) obtaining position information of each sampling point;
e3) detecting the ultrasound image data adjacent to each sampling point;
e4) calculating adjacencies between each sampling point and the detected ultrasound image data; and
e5) forming the arbitrary M-mode image data of each sampling point by using the calculated adjacencies.

5. A method of displaying an arbitrary ultrasound image by using an ultrasound diagnostic system including a probe for acquiring ultrasound signals from a plurality of scan lines, an image signal processor, a scan converter, an input unit, an arbitrary M-mode processor and a display unit, the method comprising the steps of:
a) obtaining the ultrasound signals from the probe;
b) converting the ultrasound signals into an ultrasound image data by the image signal processor;
c) converting the ultrasound image data into a B-mode image by the scan converter;
d) displaying the B-mode image by the display unit;
e) receiving an arbitrary M-mode scan line on the B-mode image from the input unit;
f) forming an arbitrary M-mode image data corresponding to the arbitrary M-mode scan line by the arbitrary M-mode processor; and
g) displaying the arbitrary M-mode image data at the display unit.

6. The method of Claim 5, wherein the step f) includes the steps of:
f1) sampling a predetermined number of points on the arbitrary M-mode scan line;
f2) setting virtual lines that pass on each sampling point and face toward the probe and obtaining position information of each sampling point;
f3) selecting adjacent points from the ultrasound image data for each sampling data, wherein the adjacent points are adjacent to said each sampling point and are positioned on the arbitrary M-mode scan lines;
f4) calculating adjacencies between said each sampling point and the adjacent points; and
f5) forming the arbitrary M-mode image data of said each sampling point by reflecting the calculated adjacencies to the ultrasound image data of the adjacent points.

7. The method of Claim 6, wherein the step f2) includes the steps of:
f21) calculating angles between the virtual lines and a vertical scan line of the probe; and
f22) calculating distances from the probe to each of the sampling points.

8. The method of Claim 6, wherein the step f4) includes the steps of:
f41) calculating angles between a vertical scan line of the probe and the arbitrary M-mode scan lines that have adjacent points; and
f42) calculating distances from the probe to each of the adjacent points.
